# EUROPEAN PATENT APPLICATION

(11) **EP 3 372 273 A1**
(43) Date of publication of application: **12.09.2018**
(21) Application number: 16861782.7
(22) Date of filing: 02.11.2016
(51) Int. Cl.: A61M 35/00, A45D 34/04

(54) **BEAUTY AND HEALTH DEVICE AND CARTRIDGE**

(30) Priority: 02.11.2015 JP 2015215532
(71) Applicant: Shiseido Company, Ltd., Chuo-ku, Tokyo 104-0061 (JP)
(72) Inventor: NASU, Mieko, Yokohama-shi Kanagawa 224-8558 (JP); TAKAHASHI, Akiko, Yokohama-shi Kanagawa 224-8558 (JP); NAGOSHI, Masahiko, Yokohama-shi Kanagawa 224-8558 (JP); TAKATA, Motoki, Yokohama-shi Kanagawa 224-8558 (JP)
(74) Representative: Scholz, Volker
(86) International application number: PCT/JP2016/004796
(87) International publication number: WO 2017/077709

(57) **Abstract**

To provide a health and beauty device capable of applying a cosmetic material or the like while heating or cooling a body surface of a user, which also enables exchange of a cosmetic material to that of a different type in a simple manner when all of the cosmetic material has been expended, or according to the target of application or the user's preference.

[Constitution]

A health and beauty device includes: a cartridge (20) that stores a fluid content (25) which is applied to a body surface of a human body to impart at least one of a cosmetic effect and a health effect, equipped with an outlet (26) from which the content (25) flows out, and a feeding portion (23) that causes the content to flow out from the outlet (26) by an externally applied force; and a housing (10) that removably accommodates the cartridge (20). A coating portion (12) having a discharge outlet (12a) aligned with the outlet (26) of the cartridge (20) and a coating surface (12b) for applying the content (25) that flows out of the outlet (26) and the discharge outlet (12a) to the body surface of the human body; a temperature operation portion (14) incorporated in the coating portion (12) that heats or cools the coating surface (12b) by receiving electric power; and a battery housing portion (10b) that houses a battery (40) are provided in the housing (10).

## Description

### [Technical Field]

The present invention is related to a health and beauty device, and in greater detail to a health and beauty device capable of applying a cosmetic material, a pharmaceutical agent, or the like while warming or cooling a body surface of a user.

The present invention also relates to a cartridge that constitutes a health and beauty device such as that described above.

### [Background Art]

Conventionally, in cosmetic material application containers for applying cosmetic materials to a body surface, there is known an applicator that employs a heat storage material for a coating portion for spreading a cosmetic material to cause heat transfer at the body surface (Patent Document 1), as well as a cosmetic material coating applicator having a member with a high cooling effect incorporated therein to apply a cosmetic material while cooling the body surface (Patent Document 3). Meanwhile, there is also known a beauty device having a heating head with the purpose of applying heat to and massaging a skin surface, which is taken out separately to spread a cosmetic material which is coated on lips while applying heat, to obtain a massaging effect (Patent Document 2).

### [Background Art Documents]

### [Patent Documents]

[Patent Document 1]
   Japanese Patent No. 5463337
[Patent Document 2]
   Japanese Unexamined Patent Publication No. 2013-162812
[Patent Document 3]
   Japanese Patent No. 5681707

### [Summary of the Invention]

### [Technical Problem]

However, in the aforementioned heating massage beauty device, it is necessary to prepare the cosmetic material separately, coat the cosmetic material, and then spread it with the applicator thereafter, and it is not possible to discharge and coat the cosmetic material simultaneously. In addition, in the conventional cosmetic material container and the conventional applicator, the content which is stored in the container is limited to one type for which the applicator is mounted, and when all of the content has been expended, the cosmetic material coating container having the applicator mounted thereon is discarded along with the applicator, resulting in such cosmetic material coating containers being perceived as expensive products. Further, there are no conventional heating beauty devices having heating units that simultaneously discharge, apply, and heat cosmetic materials on the skin simultaneously, and are capable of exchanging cosmetic materials, which are the contents, with a simple mechanism.

The present invention has been developed in view of the foregoing circumstances. It is an object of the present invention to provide a health and beauty device capable of applying a cosmetic material or the like while heating or cooling a body surface of a user, which also enables exchange of a cosmetic material to that of a different type when all of the cosmetic material has been expended, or even if the cosmetic material still remains, in a simple manner, according to the target of application or the user's preference.

Another object of the present invention is to provide a cartridge that constitutes a health and beauty device such as that described above.

### [Solution to Problem]

A first health and beauty device according to the present invention comprises:
a cartridge equipped with a storage portion that stores a fluid content which is applied to a body surface of a human body to impart at least one of a cosmetic effect and a health effect, an outlet from which the content flows out, and a feeding portion that causes the content to flow out from the outlet by an externally applied force;
a housing that removably accommodates the cartridge,
a coating portion provided in a part of the housing, having a discharge outlet aligned with the outlet of the cartridge accommodated in the housing and a coating surface for applying the content that flows out of the outlet and the discharge outlet to the body surface of the human body;
a temperature operation portion for heating or cooling the coating surface by receiving electric power; and
a power source unit for supplying the electrical power.

Note that the above "fluid content" refers to a substance which is constituted by a liquid and which does not retain the outer shape thereof unless it is contained in a container such as the aforementioned cartridge or the like. Examples of such substances also include both semisolids constituted by a paste that cannot retain the outer shape thereof unless contained in a container, and semisolids constituted by a paste or a jelly which is capable of retaining the outer shape thereof but easily deform when subjected to a certain degree of external force.

The present specification will refer to the device of the present invention as a "health and beauty device", even if the device has only one of a cosmetic effect and a health effect described above.

A second health and beauty device according to the present invention comprises:
a cartridge equipped with a storage portion that stores content which is applied to a body surface of a human body to impart at least one of a cosmetic effect and a health effect, a heating unit that heats the content to impart fluidity thereto, a coating portion having a discharge outlet from which the content flows out and a coating surface for applying the content that flows out of the discharge outlet to the body surface of the human body, and a feeding portion that causes the content to flow out from the discharge outlet by an externally applied force;
a housing that removably accommodates the cartridge,
a temperature operation portion for heating or cooling the coating surface by receiving electric power; and
a power source unit for supplying the electrical power. Note that the heating unit may also be used as the temperature operation portion.

Here, the aforementioned content is preferably a cosmetic material. In addition, although such a cosmetic material is not particularly limited, it is desirable for the cosmetic material to be that which is a body surface such as a face, a scalp, a body part and the like, particularly a lip cosmetic material and an eye cosmetic material. Alternatively, the aforementioned content may be a pharmaceutical agent.

Meanwhile, in the health and beauty device of the present invention, it is desirable for the feeding portion of the cartridge to comprise a rotatable operating portion which rotates upon receipt of an external force and a moving body that moves within the cartridge in a direction that approaches the outlet or the discharge outlet by the rotatable operating portion being rotated. Alternatively, the feeding portion of the cartridge may include a knock operating portion which moves forward and backward in response to an external knocking force, and a moving body that moves within the cartridge in a direction that approaches the outlet or the discharge outlet by operation of the knock operating portion. As a further alternative, the feeding portion of the cartridge may be a flexible member that constitutes at least a portion of the outer shell of the cartridge and applies pressure to the stored content by deforming under a an externally applied squeezing force. Further, the feeding portion is not limited to those described above, and other known feeding portions may be employed.

In the health and beauty device of the present invention, it is preferable for the temperature operation portion to have a planar portion provided adjacent to the interior of the coating portion.

In addition, in the health and beauty device of the present invention, it is desirable for both the cartridge and the power source unit (for example, a battery) to be formed as approximate rod shapes, and for the cartridge and the power source unit to be arranged in series with each other in the longitudinal direction. Note that the expression "in series with each other in the longitudinal direction" above does not necessarily mean that they are coaxially aligned, but the longitudinal directions of the two are substantially parallel to each other, and the two components are arranged in the longitudinal direction means that the two components at least partially overlap when projected in the longitudinal direction.

Alternatively, in the health and beauty device of the present invention, it is desirable for both the cartridge and the power source unit to be formed as approximate rod shapes, and for the cartridge and the power source unit to be arranged parallel to each other with a gap therebetween in a direction that intersects with the respective longitudinal directions thereof.

As a further alternative, in the health and beauty device of the present invention, it is desirable for both the cartridge and the power source unit to be formed as approximate rod shapes, and for the power source unit to be arranged at a position remote from the cartridge in the longitudinal direction of the cartridge, oriented such that the longitudinal direction of the power source unit intersects the longitudinal direction of the cartridge. Note that the expression "the longitudinal direction of the power source unit intersects with the longitudinal direction of the cartridge" above does not necessarily mean that the central axes of the two intersect each other, but that when the cartridge is projected in the longitudinal direction, the cartridge and at least part of the power source unit intersect each other.

Meanwhile, a first cartridge according to the present invention is a cartridge which is removably accommodated in a housing that constitutes a health and beauty device, and is characterized by:
storing a fluid content which is applied to a body surface of a human body to impart at least one of a cosmetic effect and a health effect; and comprises:
an outlet from which the content flows out; and
a feeding portion that causes the content to flow out from the outlet by an externally applied force.

In addition, a second cartridge according to the present invention is a cartridge which is removably accommodated in a housing that constitutes a health and beauty device, and is characterized by comprising:
a storage portion that stores content which is applied to a body surface of a human body to impart at least one of a cosmetic effect and a health effect;
a heating unit that heats the content to impart fluidity thereto;
a coating portion having a discharge outlet from which the content flows out and a coating surface for applying the content that flows out of the discharge outlet to the body surface of the human body; and
a feeding portion that causes the content to flow out from the discharge outlet by an externally applied force.

### [Advantageous Effects of the Invention]

The first health and beauty device according to the present invention is basically constituted by: the cartridge that stores the fluid content which is applied to a body surface of a human body to impart at least one of a cosmetic effect and a health effect, equipped with the outlet from which the content flows out, and the feeding portion that causes the content to flow out from the outlet by an externally applied force; and the housing that removably accommodates the cartridge. Further, a mechanism for heating or cooling a coating surface is provided. Therefore, this health and beauty device easily enables simultaneous discharge and application of a cosmetic material or the like. In addition, this health and beauty device enables different types of cosmetic materials or the like to be easily exchanged according to the target of application or the user's preference and utilized.

In addition, the second health and beauty device according to the present invention is basically constituted by: the cartridge equipped with the storage portion that stores the content which is applied to a body surface of a human body to impart at least one of a cosmetic effect and a health effect, the heating unit that heats the content to impart fluidity thereto, the coating portion having the discharge outlet from which the content flows out and the coating surface for applying the content that flows out of the discharge outlet to the body surface of the human body, and a feeding portion that causes the content to flow out from the discharge outlet by an externally applied force; and the housing that removably accommodates the cartridge. Further, a mechanism for heating or cooling a coating surface is provided. Therefore, this health and beauty device easily enables simultaneous discharge and application of a cosmetic material or the like. In addition, this health and beauty device enables different types of cosmetic materials or the like to be easily exchanged according to the target of application or the user's preference and utilized.

### [Brief Description of the Drawings]

[Figure 1] A perspective view that illustrates a health and beauty device according to a first embodiment of the present invention
[Figure 2] An exploded perspective view that illustrates a portion of the components of the health and beauty device of Figure 1
[Figure 3] A front view that illustrates an example of the manner of use of the health and beauty device of Figure 1
[Figure 4] A perspective view that illustrates a health and beauty device according to a second embodiment of the present invention
[Figure 5] An exploded perspective view that illustrates a portion of the components of the health and beauty device of Figure 4
[Figure 6] A perspective view that illustrates a health and beauty device according to a third embodiment of the present invention
[Figure 7] An exploded perspective view that illustrates a portion of the components of the health and beauty device of Figure 6
[Figure 8] A perspective view that illustrates a health and beauty device according to a fourth embodiment of the present invention
[Figure 9] An exploded perspective view that illustrates a portion of the components of the health and beauty device of Figure 8
[Figure 10] A side sectional view that illustrates a health and beauty device according to a fifth embodiment of the present invention
[Figure 11] A perspective view that illustrates a heater in the configuration of Figure 10
[Figure 12] A sectional view that illustrates a portion around the periphery of the heater of Figure 11
[Figure 13] A perspective view that illustrates another example of a heater
[Figure 14] A sectional view that illustrates a portion around the periphery of the heater of Figure 13
[Figure 15] A side sectional view that illustrates a health and beauty device according to a sixth embodiment of the present invention
[Figure 16] A perspective view that illustrates an inner plug portion in the configuration of Figure 15
[Figure 17] A perspective view that illustrates another example of an inner plug portion
[Figure 18] A perspective view that illustrates a coating head in the configuration of Figure 15
[Figure 19] A side sectional view of the coating head of Figure 18
[Figure 20] A partially sectional perspective view that illustrates another example of a coating head
[Figure 21] An exploded perspective view that illustrates yet another example of a coating head
[Figure 22] An exploded perspective view that illustrates still yet another example of a coating head
[Figure 23] A side sectional view that illustrates a health and beauty device according to a seventh embodiment of the present invention

### [Description of Embodiments]

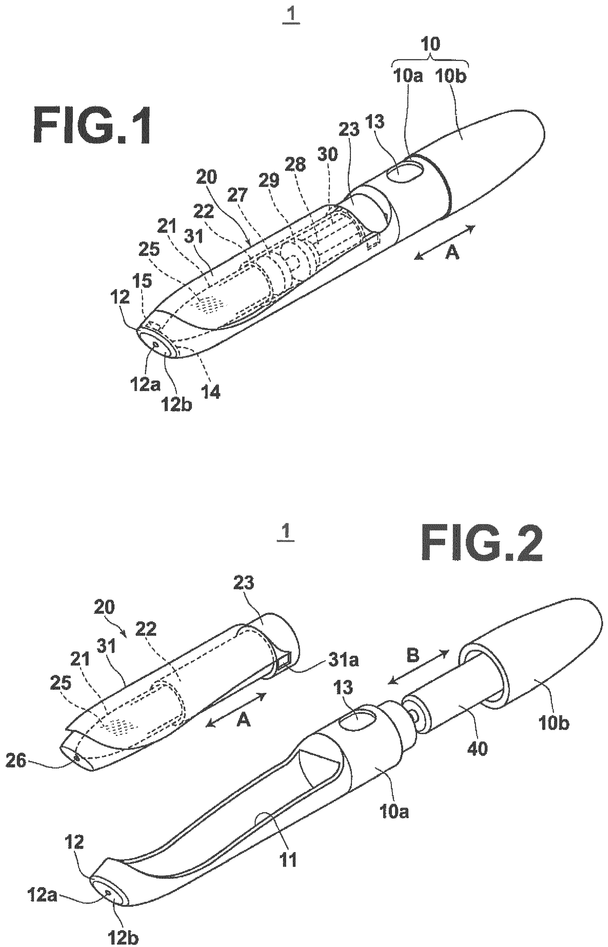

Embodiments of the present invention will now be described in detail with reference to the attached drawings. Figure 1 is a perspective view that illustrates a health and beauty device 1 according to a first embodiment of the present invention, and Figure 2 is a perspective view that illustrates the health and beauty device 1 in a disassembled state. As illustrated in these figures, the health and beauty device 1 of the present embodiment includes a housing 10 constituted by a main body 10a and a lid 10b, and a cartridge 20 which is removably accommodated in the housing 10.

The cartridge 20 is formed in an approximate rod shape from synthetic resin, for example, and includes a storage chamber 21 having an interior space arranged at a leading end portion (the left end in the figure), a cylinder chamber 22 that communicates with the back end of the storage chamber 21, and a rotatable operating portion 23 arranged at the side of the back end of the cylinder chamber 22. The above parts are arranged in the order described above along the longitudinal direction (the direction of the arrow A in the figure) of the approximately rod shaped cartridge 20.

A content 25 of fluid which is applied to a body surface of a human body impart at least one of a cosmetic effect and a health effect is stored in the storage chamber 21. In the present embodiment, for example, a lip plumper, which is a lip cosmetic material, is stored as the content 25. An outlet 26, through which the content 25 flows out, is formed at the leading end of the cartridge 20, that is, at the leading of the storage chamber 21.

The cylinder chamber 22 is a cylindrical portion having an interior space that extends in the longitudinal direction of the cartridge 20, and a piston 27 is provided in the interior space as a moving member. The front end of a threaded rod 28 having a male threaded portion formed on the peripheral surface thereof is connected to the piston 27. The threaded rod 28 is in threaded engagement with and inserted through a female threaded aperture formed at the center of a fixed holding portion 29. The back end portion of the threaded rod 28 is formed as a round bar shape on which a male threaded portion is not formed, and this portion is rotatable with a rotary holding portion 30 with respect to the rotary holding portion 30, and is held so as to be movable relative thereto in the axial direction of the threaded rod 28. The rotary holding portion 30 is configured to be rotatable together with the rotatable operating portion 23.

The configuration formed by the elements 27 through 30 is a rotary feeding mechanism which is widely applied to lipstick containers and the like and constitutes a feeding portion for causing the contents 25 to flow out from the outlet 26 in the present invention. That is, when the rotatable operating portion 23 is rotated in a predetermined direction by an external force, the threaded rod 28 is rotated via the rotary holding portion 30, and the threaded rod 28 advances due to the threaded rod 28 and the female threaded aperture of the fixed holding portion 29 being in threaded engagement, to move the piston 27 toward the side of the leading end. When the piston 27 moves in this manner, the content 25 in the storage chamber 21 is pressed, and a portion thereof flows out from the outlet 26.

Note that a ratchet mechanism may be incorporated in the rotatable operating portion 23 or the rotary holding portion 30 such that the rotatable operating portion 23 is rotatable only in a direction that causes the piston 27 to advance, and not rotatable in a direction that causes the piston 27 to retreat.

A cover 31 is integrally attached to the storage chamber 21, the cylinder chamber 22, and the rotatable operating portion 23. The cover 31 has a pair of engagement claws 31a (only one is illustrated in Figure 2) at the back end portion. The engaging claws 31a of the cover 31 and the portions in the vicinities of the bases thereof are elastically deformable.

A majority of the main body 10a and the lid 10b that constitute the housing 10 is also of synthetic resin, for example. As illustrated in Figure 2, the lid 10b is integrated with the main body 10a by threaded engagement, for example. The lid 10 b and a portion of the main body 10a with which the lid 10b is threadedly engaged constitute a battery housing portion for accommodating a battery 40. As illustrated in Figure 2, after inserting a portion of a + (plus: anode) side into the main body 10a, the battery 40 is covered by the lid 10b at a - (minus: cathode) side, and the lid 10b is threadedly engaged with the main body 10a to accommodate the battery 40 in the housing 10.

A rod shaped dry battery such as a triple A battery or a double A battery, for example, is employed as the battery 40. In the present embodiment, the cartridge 20 and the battery 40 are arranged in series such that the longitudinal direction (the direction denoted by the arrow B) of the rod shaped battery 40 and the longitudinal direction (the direction denoted by the arrow A) of the similarly rod shaped cartridge 20 are aligned.

The main body 10a of the housing 10 has a recess 11 for accommodating the cartridge 20, a coating portion 12 disposed at the leading end portion, and a switch 13. When the cartridge 20 is accommodated in the housing 10, the cartridge 20 is first stored in the recess 11 from the side of the leading end, and then the pair of engaging claws 31a elastically engage with engaging portions (not shown) formed in the main body 10a. Thereby, the cartridge 20 is accommodated and held in the recess 11 of the housing 10. When removing the cartridge 20 which is accommodated in the housing 10, a portion in the vicinity of the engagement claw 31a of the cover 31 is pushed inwardly to release the engagement, and the cartridge 20 is removed by withdrawing the cartridge 20 from the recess 11.

The coating portion 12 is formed by stainless steel for example, and a discharge outlet 12a is formed in the center portion thereof. The surface 12b of this coating portion 12 is a coating surface for applying the content 25 to the body surface of a user. The discharge outlet 12a is provided at a position which is aligned with the outlet 26 of the cartridge 20 when the cartridge 20 is accommodated in the recess 11. Further, a heater 14 is incorporated adjacent to the inner side of the coating portion 12 as a temperature operation portion. A ceramic resistor or a metal having a planar heating unit or the like may be favorably employed as the heater 14, but the heater 14 is not limited thereto, and a Ni chrome wire in a wound state or the like may alternatively be employed. In the case that the heater 14 is installed adjacent to the inside of the coating portion 12 as described above, it is preferable from the viewpoint of thermal efficiency to dispose the heater 14 so that heating is performed on the back surface of the surface 12b, which is a coating surface. A temperature sensor 15 constituted by a thermistor or the like for example, is provided in the vicinity of the heater 14.

Note that the coating portion 12 may be formed by a metal different from the above stainless steel, for example, aluminum. The coating portion 12 may also be formed by a material other than metal, such as silicone, a ceramic, a synthetic resin or the like. Further, the surface 12b of the coating portion 12 may be a smooth surface, a surface having fine grooves or recesses formed therein, or a roughened surface having fine protrusions and recesses formed thereon. The shape of the surface 12b of the coating portion 12 as a whole may be a flat surface, a convex surface, or a concave surface.

A control circuit (not shown) formed by a printed circuit board or the like is provided in the main body 10a of the housing 10. This circuit board is electrically connected to the battery 40, the switch 13, the heater 14, and the temperature sensor 15 via wiring (not shown). A switch of the type that switches between an ON state in which direct current from the battery 40 is supplied to the heater 14 and an OFF state in which the supply of current is cut off each time that it is pressed, for example, is employed as the switch 13.

When the switch 13 is set to the ON state, the heater 14 which receives the current generates heat, and thereby the surface 12 b of the coating portion 12 is heated. The temperature of the surface 12b of the heated coating portion 12 is detected by the temperature sensor 15. Temperature detection signals which are output by the temperature sensor 15 are output to the control circuit, and the control circuit controls the supply of current to the heater 14 based on the temperature detection signals to maintain the temperature of the surface 12b of the coating portion 12 at a predetermined set temperature. This set temperature is set to be somewhat higher than common body temperature, 40°C or the like, for example.

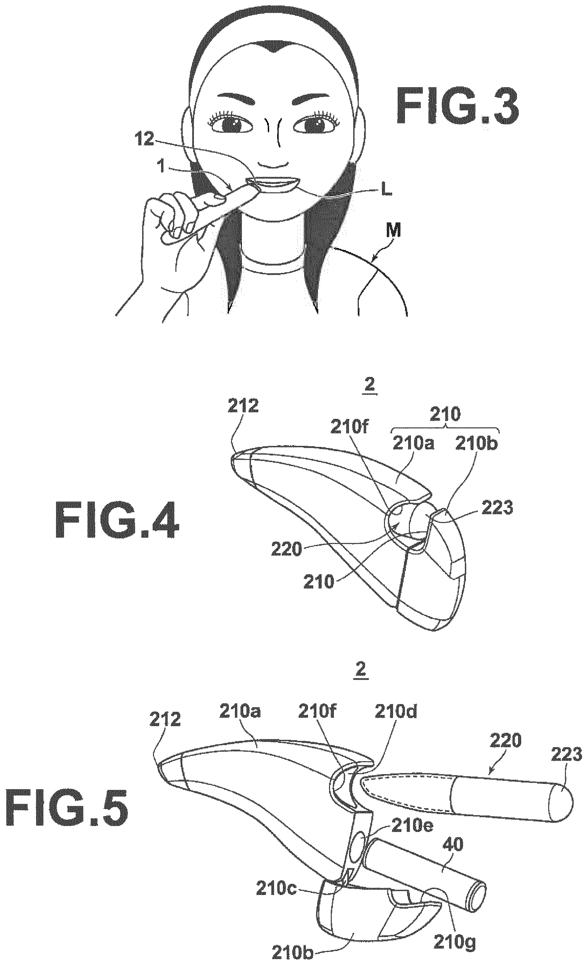

Next, a method of use of the health and beauty device 1 having the configuration described above will be described with reference to Figure 3. A user M holds the health and beauty device 1 in one hand, turns on the switch 13, and then operates the rotatable operating portion 23 to discharge a desired amount of content 25 from the storage chamber 21 of the cartridge 20. The content 25 flows out and adheres onto the surface 12b of the coating portion 12 through the outlet 26 of the cartridge 20 and the discharge outlet 12a which is aligned with the outlet 26.

Next, the user M brings the leading end side of the health and beauty device 1 close to themselves, and applies the content (lip plumper) 25 which is adhered to the surface 12b of the coating portion 12 to their lips L. At this time, since the switch 13 is turned on and current is being supplied to the heater 14, the temperature of the coating portion 12 is maintained in the vicinity of 40°C as described above, for example. Therefore, when applying the content 25 to the lips L, the user M experiences a distinctively warm feeling of application from the content 25 and the coating portion 12. Such a feeling of application is generally pleasant for the user M and promotes use of the health and beauty device 1.

Also, as the content 25 softens due to being heated, the content 25 will be applied favorably into wrinkles, even if wrinkles are present on the lips L. In addition, as the lip L is warmed, blood circulation also improves, so the appearance of the lip L will also improve. Furthermore, particularly in the case that the content 25 is a pharmaceutical agent, penetration into the skin is also promoted, and an effect of improving a medicinal effect is also obtained.

Note that in the case that the user M becomes accustomed to using the health and beauty device 1, the user M may first contact the coating portion 12 of the health and beauty device 1 to the lip L, turn on the switch 13, and then operate the rotatable operating portion 23.

When application of the content 25 in the manner described above is repeated many times and all of the content 25 in the storage chamber 21 is expended, the cartridge 20 is exchanged for a new one, that is, another cartridge in which a predetermined amount of content 25 is stored in the storage chamber 21 thereof. When exchanging the cartridge 20, the operation of removing the cartridge 20 from the housing 10 and the operation of accommodating the new cartridge 20 in the housing 10 are performed in the manners described above.

When all of the content 25 within the cartridge 20 is expended, if only the cartridge 20 is exchanged for a new one in this manner, the housing 10 is still utilizable as is, which is economical. Also, the operation to exchange the cartridge 20 can be performed extremely simply, unlike an operation to supplement content in a storage portion of a health and beauty device.

Next, a second embodiment of the present invention will be described. Figure 4 is a perspective view that illustrates a health and beauty device 2 according to the second embodiment of the present invention, and Figure 5 is a perspective view that illustrates the health and beauty device 2 in a partially disassembled state. Note that in Figure 4 and the figures thereafter, elements which are the same as those of the first embodiment which was described previously are denoted by the same reference numerals, and descriptions thereof will be omitted unless particularly necessary. As illustrated in Figure 4 and Figure 5, the health and beauty device 2 of the present embodiment includes a housing 210 constituted by a main body 210a and a lid 210b, and a cartridge 220 which is removably accommodated in the housing 210. The cartridge 220 has the same basic configuration as the cartridge 20 described above.

The lid 210b is swingably attached to the main body 210a via a hinge 210c to open and close a rear portion of the main body 210a. The main body 210a is formed in a substantially trapezoidal box shape and has a cartridge housing portion 210d for accommodating the cartridge 220 and a battery housing portion 210e for accommodating the battery 40. The main body 210a and the lid 210b respectively have cutout portions 210f and 210g.

When the lid 210b is in a state in which the rear portion the main body 210a is open as illustrated in Figure 5, it is possible to accommodate the cartridge 220 in the cartridge housing portion 210d and to accommodate the battery 40 in the battery housing portion 210e. Removal of the cartridge 220 from the cartridge housing portion 210d and removal of the battery 40 from the battery housing portion 210e are performed in the same manner with the lid 210b in a state in which the rear portion of the main body 210a is open.

When the health and beauty device 2 is utilized, the lid 210b is in a state in which the rear portion of the main body 210a is closed, as illustrated in Figure 4. In this state, a rotatable operating portion 223 of the cartridge 220 is positioned in the cutout portions 210f and 210g, and is exposed to the exterior. Therefore, the content can be caused to flow out from the coating portion 212 by operating the rotatable operating portion 223 in a simple manner, and be applied to lips or the like.

Note that although a discharge outlet and the like of the coating portion 212 are not illustrated, the discharge outlet and the outlet of the cartridge 220 for the content may basically be formed in the same manner as in the first embodiment. Further, a heater which is incorporated in the coating portion 212 as a temperature operation portion incorporated in the coating portion 212 is also omitted from the figures. However, a heater similar to the heater 14 of the first embodiment is provided in the present embodiment as well. This applies to the third and fourth embodiments to be described below as well.

As clearly illustrated in Figure 4 and Figure 5, in the present embodiment, both the cartridge 220 and the battery 40 are formed as approximate rod shapes. The cartridge 220 and the battery 40 are arranged parallel to each other with a gap therebetween in a direction that intersects with the respective longitudinal directions thereof.

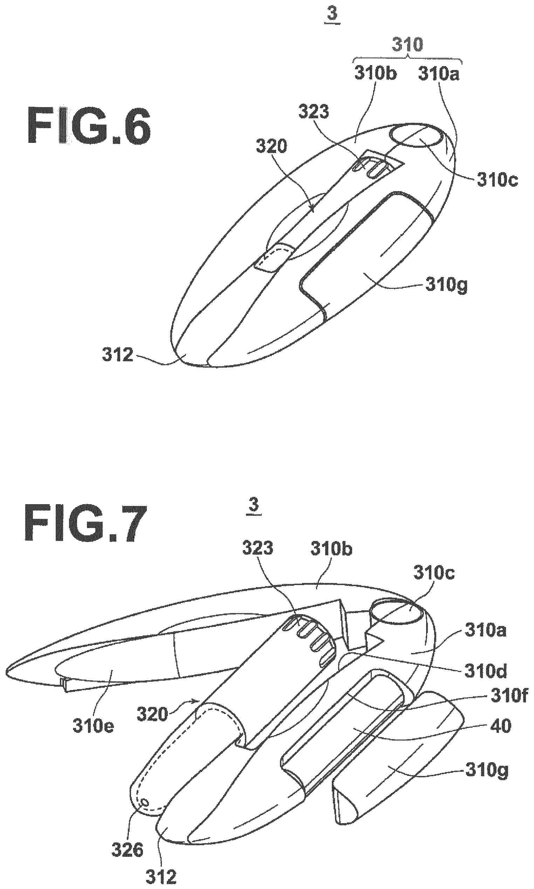

Next, a third embodiment of the present invention will be described. Figure 6 is a perspective view that illustrates a health and beauty device 3 according to the third embodiment of the present invention, and Figure 7 is a perspective view that illustrates the health and beauty device 3 in a partially disassembled state. As illustrated in these figures, the health and beauty device 3 of the present embodiment has a housing 310 constituted by a right housing 310a and a left housing 310b, and a cartridge 320 which is removably accommodated in the housing 310. The cartridge 320 has the same basic configuration as the cartridge 20 described above.

The right housing 310a and the left housing 310b are rotatable about a pivot shaft 310c as an axis, and are capable of being in a closed state as illustrated in Figure 6 with the cartridge 320 sandwiched therebetween, and in a widely spread apart open state of as illustrated in Figure 7. Cartridge accommodating recesses 310d and 310e are respectively formed in the right housing 310a and the left housing 310b. The element denoted by reference numeral 312 in Figure 6 and Figure 7 is a coating portion of the housing 310, and the element denoted by reference number 326 in Figure 7 is a content outflow outlet of the cartridge 320.

When accommodating the cartridge 320 in the housing 310, a portion of the cartridge 320 is accommodated in the cartridge accommodating recess 310d or 310e with the right housing 310a and the left housing 310b in an open state. Thereafter, when the right housing 310a and the left housing 310b are closed, the cartridge 320 is accommodated and held in the housing 310 in a state in which portions thereof are respectively within the two cartridge accommodating recesses 310d and 310e. The state in which the right housing 310a and the left housing 310b are closed is maintained by latch means or the like (not shown). It is possible to remove the cartridge 320 which is accommodated in the housing 310 in this manner from the housing 310 if the right housing 310a and the left housing 310b are opened in the manner described above.

The right housing 310a is provided with a battery housing portion 310f, and the battery 40 is accommodated in the battery housing portion 310f. A lid 310g is fitted on the battery housing portion 310f. The right housing 310a and the left housing 310b are shaped so as to partially expose the cartridge 320 held therebetween. Thereby, a rotatable operating portion 323 of the exposed cartridge 320 can be operated from the exterior.

When the health and beauty device 3 is utilized, the right housing 310a and the left housing 310b are closed and the cartridge 320 is held therebetween, as illustrated in Figure 6. In this state, content can be caused to flow out from the coating portion 312 by operating the rotatable operating portion 323, which is exposed as described above, and be applied to lips or the like.

As clearly illustrated in Figure 6 and Figure 7, in this embodiment, both the cartridge 320 and the battery 40 are formed in a rough rod shape. The cartridges 320 and the batteries 40 are arranged in parallel to each other with a gap therebetween in a direction intersecting the respective longitudinal directions thereof.

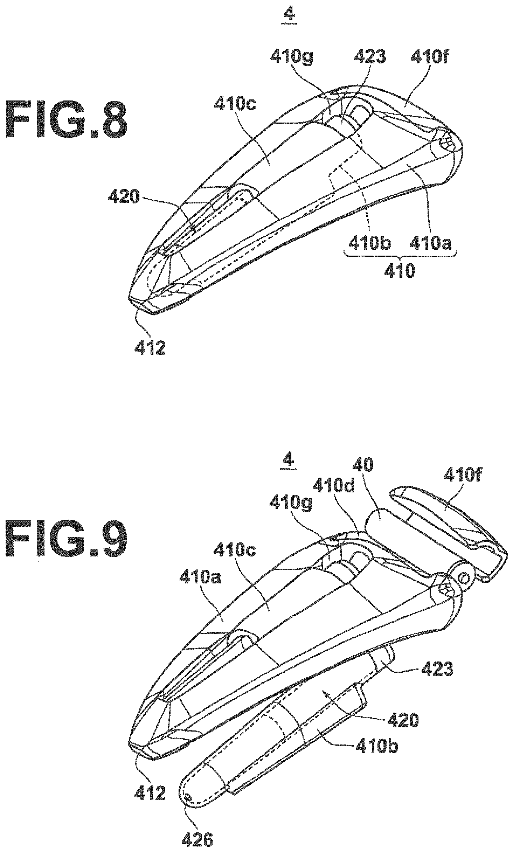

Next, a fourth embodiment of the present invention will be described. Figure 8 is a perspective view that illustrates a health and beauty device 4 according to the fourth embodiment of the present invention, and Figure 9 is a perspective view that illustrates the health and beauty device 4 in a partially disassembled state. As shown in these figures, the health and beauty device 4 of the present embodiment comprises a housing 410 constituted by a substantially triangular box shaped main body 410a and a lid 410b, and a cartridge 410 which is removably accommodated in the housing 410. The cartridge 420 has the same basic configuration as the cartridge 20 described above.

The main body 410a of the housing 410 has a shape having an opening 410g that extends in the longitudinal direction of the housing at the center in the width direction thereof, and a portion of the opening 410g is provided with a cartridge receiving portion 410c for receiving the cartridge 420. In Figure 8 and Figure 9, the element denoted by reference numeral 412 is a coating portion of the housing 410, and the element denoted by reference numeral 426 in Figure 9 is an outlet of the cartridge 420, through which content flows out.

When accommodating the cartridge 420 in the housing 410, the lid 410b is removed from the main body 410a, the cartridge 420 is placed in a cartridge receiving portion 410c from the lower side, and then the lid 410b is fitted and attached to the main body 410a from the lower side and so as to hold the cartridge 420. This attachment is made by engaging an engaging claw provided on the lid 410b with an engaging portion of the main body 410a, although not shown in the figures. The cartridge 420 which is accommodated in the housing 410 in this manner can be removed from the housing 410 by releasing the above engagement and removing the lid 410b from the main body 410a.

A battery housing portion 410d is provided at the rear end portion of the main body 410a, and the battery 40 is accommodated in the battery housing portion 410d. A lid 410f is fitted on the battery housing portion 410d.

When this health and beauty device 4 is utilized, the cartridge 420 is accommodated and held in the housing 410, as illustrated in Figure 8. In this state, a rotatable operating portion 423 of the cartridge 420 is exposed from a part of the opening 410g, as illustrated in the figure. Therefore, content can be caused to flow out from the coating portion 412 by rotationally operating the exposed rotatable operating portion 423, and be applied to lips and the like.

As clearly illustrated in Figure 8 and Figure 9, in the present embodiment, both the cartridge 420 and the battery 40 are formed as approximate rod shapes. The battery 40 is disposed at a position away from the cartridge 420 in the longitudinal direction of the cartridge 420 such that the longitudinal direction of the battery intersects with the longitudinal direction of the cartridge.

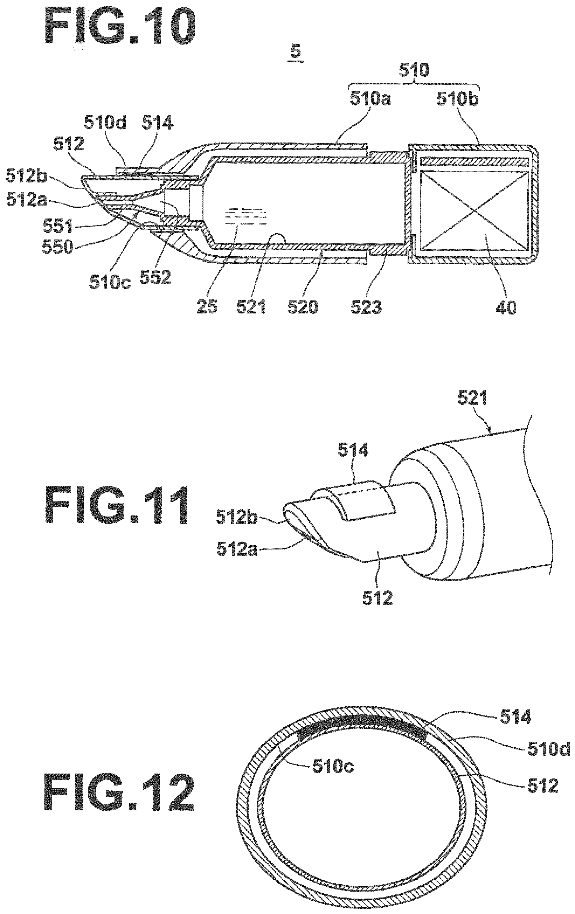

Next, a fifth embodiment of the present invention will be described. Figure 10 is a side sectional view showing a health and beauty device 5 according to the fifth embodiment of the present invention. The health and beauty device 5 of the present embodiment includes a housing 510 constituted by a main body 510a and a lid 510b, and a cartridge 520 which is removably accommodated in the housing 510. The main body 510a and the lid 510b which are both formed in a substantially cylindrical shape are illustrated in a separated state in Figure 10, but are separably coupled by parts which are not illustrated. The lid 510b constitutes a battery housing portion that houses a battery 40 as a power source unit. Meanwhile, the cartridge 520 differs from the cartridges which have been described thus far, in that the cartridge 520 itself is equipped with a coating portion for applying content.

Hereinafter, this cartridge 520 will be described in detail. The cartridge 520 includes a substantially cylindrical storage chamber 521 that stores the content 25, a rotatable operating portion 523, an inner plug portion 550 connected to the storage chamber 521, and a coating head 512 as a coating portion which is fitted on the inner plug portion 550. The rotatable operating portion 523 is coupled to a mechanism for feeding the content 25 from the storage chamber 521, that is, the mechanism constituted by the piston 27, the threaded rod 28, etc. illustrated in Figure 1. However, this mechanism is omitted from Figure 10. The inner plug portion 550 has a thin cylindrical leading end portion 551. A channel 552 that communicates with the storage chamber 521 and extends to the interior of the leading end portion 551 is formed within the inner plug portion 550.

The coating head 512 is formed by a metal having favorable thermal conducting properties such as aluminum, an aluminum alloy, and stainless steel, for example. The surface 512b of the leading end thereof is formed flat to be a coating surface for applying the content 25 to a body surface of a human body. An outlet 512a through which the content 25 flows out is formed in the surface 512b. This outlet 512a is aligned with the channel 552 in the leading end portion 551 of the inner plug portion 550. The coating head 512 is fitted on the leading end portion 551 and integrated with the inner plug portion 550. A substantially cylindrical leading end portion 510d having an inner peripheral surface 510c is formed in the main body 510a of the housing 510 described above. The coating head 512 is configured to be able to be combined with the leading end portion 510d in a state in which the outer peripheral surface thereof is in contact with the inner peripheral surface 510c. The cartridge 520 is removably accommodated in the main body 510a of the housing 510 by the coating head 512 and the leading end portion 510d being combined in this manner.

The content 25 which is stored in the storage chamber 521 flows out from the outlet 512a of the coating head 512 via the channel 552 of the inner plug portion 550, by the rotatable operating portion 523 being operated. Thereby, the content 25 which has flowed out can be applied to the body surface of the human body while the surface 512b of the coating head 512 is in contact with the body surface. A heater 514 is attached to the inner peripheral surface 510c of the leading end portion 510d of the housing main body 510a. When the cartridge 520 is set in the housing 510, the coating head 512 of the cartridge 520 is in contact with and is heated by the heater 514. The surface 512 b, which is the coating surface of the coating head 512, is heated to a predetermined temperature. Therefore, in this case as well, a user will experience a pleasant feeling of application when applying the content 25 to the body surface as described above.

Hereinafter, the heater 514 will be described in detail. Figure 11 and Figure 12 are a perspective view and a sectional view that illustrate the heater 514 and a portion of the cartridge 520 at the periphery of the heater 514, respectively. Note that the sectional view of Figure 12 shows a cross section of a plane perpendicular to the longitudinal axis of the coating head 512. As illustrated in Figure 12, the heater 514 is fixed to the inner peripheral surface 510c of the leading end portion 510d of the housing main body 510a (refer to Figure 10), and as clearly illustrated in Figure 11 as well, the entire inner surface of the heater 514 is in contact with the outer peripheral surface of the coating head 512 . Thereby, the heat generated by the heater 514 can be efficiently conducted to the coating head 512. It is desirable for the heater 514 to be elastically pressed against the coating head 512 using, for example, a cushioning material or a leaf spring to enhance the close contact properties between the heater 514 and the coating head 512.

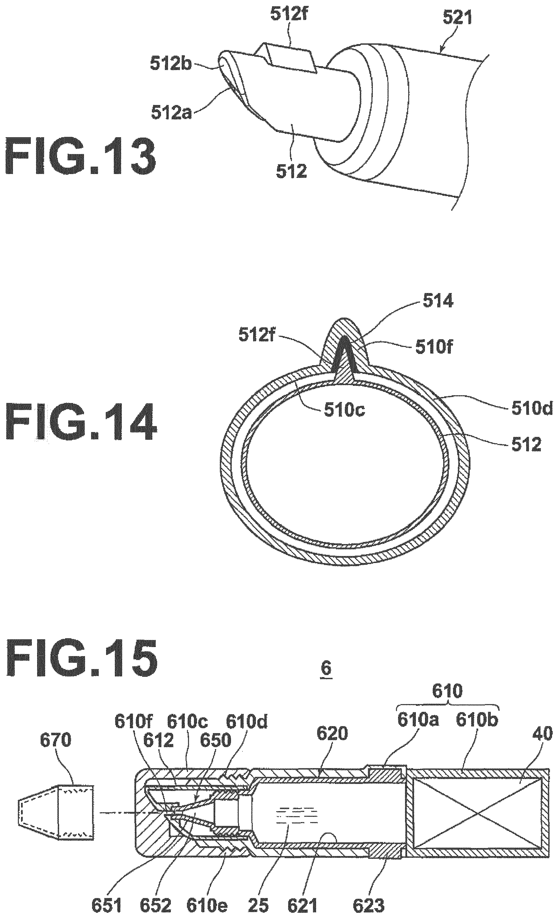

Here, another example of the heater 514 will be described. Figure 13 and Figure 14 are a perspective view and a sectional view that illustrate another example of the heater 514 and a portion of the cartridge 520 at the periphery of the heater 514, respectively. Note that the sectional view of Figure 14 shows a cross section of a plane perpendicular to the longitudinal axis of the coating head 512. As illustrated in these figures, a convex portion 510f with an inverted V shaped cross section that extends in the longitudinal direction of the leading end portion 510d is formed on the leading end portion 510d of the housing main body 510a (refer to Figure 10). The heater 514 is also formed to have an inverted V shaped cross section and is fixed to the inner surface of the convex portion 510f. Meanwhile, the coating head 512 is provided with a projecting portion 512f having a substantially triangular prism shape (that is, a tapered cross sectional shape) capable of entering the interior of the heater 514 having the V shaped cross section. Thereby, the close contact properties between the heater 514 and the coating head 512 can be enhanced in this example as well.

Next, a sixth embodiment of the present invention will be described. Figure 15 is a side sectional view that illustrates a health and beauty device 6 according to the sixth embodiment of the present invention. The health and beauty device 6 of the present embodiment includes a housing 610 constituted by a main body 610a and a lid 610b, and a cartridge 620 which is removably accommodated in the housing 610. The main body 610a and the lid 610b, both of which are formed as substantially cylindrical shapes, are shown in a state in which they are simply linked in Figure 15. However, these two components are configured to be separable in order to exchange the cartridge 620 or the battery 40 as necessary. The lid 610b constitutes a battery housing portion that houses the battery 40 as a power source unit therein. A coating head 612 is fixed to the main body 610a as a coating portion. More specifically, a leading end portion 610d having a substantially cylindrical shape is formed in the main body 610a, and the coating head 612 is fixed inside the leading end portion 610d.

Hereinafter, the above cartridge 620 will be described in detail. The cartridge 620 has a substantially cylindrical storage chamber 621 that stores the content 25, a rotatable operating portion 623, and an inner plug portion 650 which is connected to the storage chamber 621. The rotatable operating portion 623 is coupled to a mechanism for feeding the content 25 from the storage chamber 621, that is, the mechanism constituted by the piston 27, the threaded rod 28, etc. illustrated in Figure 1. However, this mechanism is omitted from Figure 15. The inner plug portion 650 has a leading end portion 651 with a thin cylindrical portion. A channel 652 that communicates with the storage chamber 621 and extends to the interior of the leading end portion 651 is formed within the inner plug portion 650. The cartridge 620 having the configuration described above is removably accommodated in the main body 610a of the housing 610.

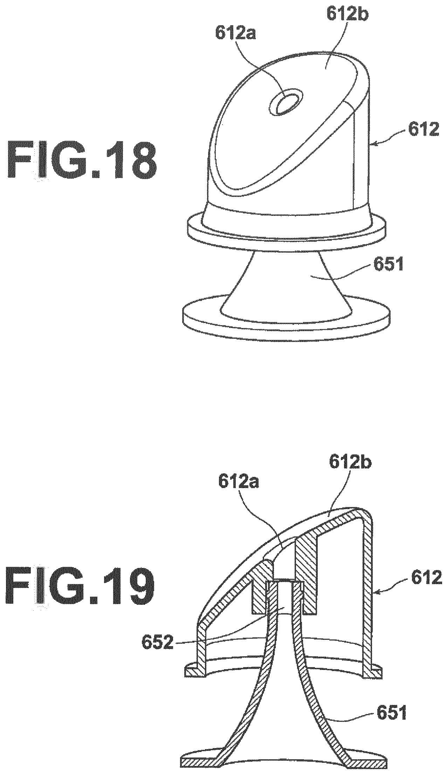

Meanwhile, the coating head 612 which is fixed to the main body 610a is formed by a metal having favorable thermal conducting properties such as aluminum, an aluminum alloy, and stainless steel, for example. Figure 18 and Figure 19 illustrate the outer shape and the side sectional shape of the coating head 612, respectively. As clearly illustrated in Figure 19, the leading end portion 651 of the inner plug portion 650 of the cartridge 620 is inserted into the cylindrical portion inside the coating head 612. The surface 612b at the leading end of the coating head 612 is formed flat so as to be a coating surface for applying the content 25 to a body surface of a human body. A discharge outlet 612a through which the content 25 flows out is formed in the surface 612b. When the cartridge 620 is set in the housing 610, the discharge outlet 612a communicates with the channel 652 in the leading end portion 651 of the inner plug portion 650. It is desirable for a sealing member constituted by a contact ring, an O ring, or the like to be provided between the leading end portion 651 and the cylindrical portion of the coating head 612, in order to prevent the channel 652 within the leading end portion 651 from communicating with a space other than the discharge outlet 612a of the coating head 612. A heater 14 similar to that illustrated in Figure 1 is provided on the side of the back surface of the surface 612b, in close contact with the back surface. When the cartridge 620 is accommodated in the main body 610a of the housing 610 as illustrated in Figure 15, the inner plug portion 650 is integrated with the coating head 612. The position of the cartridge 620 in the longitudinal direction thereof with respect to the coating head 612 (the position in the horizontal direction in Figure 15) is defined, for example, by bringing a part of the cartridge 620 into contact with ribs (not shown) in the main body 610a.

A threaded cap 610c is attachable to the main body 610a of the housing 610. The threaded cap 610c is formed in a cylindrical shape with one closed end. The threaded cap 610c is attached to the cartridge 620 by threading female threads formed on the open end 610e thereof onto a male threaded portion formed on the leading end portion 651 of the cartridge 620. In addition, a columnar protrusion 610f is provided on the inner surface of the closed end of the threaded cap 610c. In a state in which the threaded cap 610c is attached to the housing 610, the protrusion 610f enters the channel 652 of the leading end portion 651 of the inner plug portion 650. Thereby, the channel 652 is maintained in hermetically sealed state with respect to the exterior of the cartridge 620.

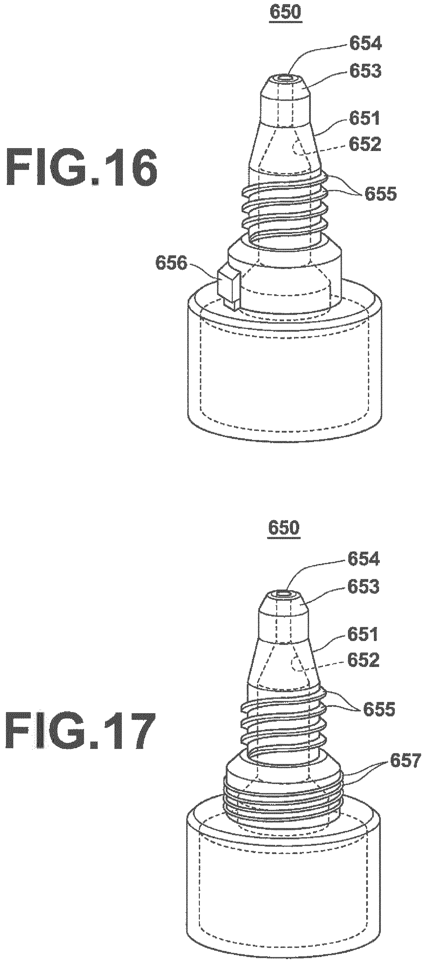

Here, the inner plug portion 650 which is provided at the side of the leading end of the cartridge 620 will be described in detail with reference to Figure 16. Figure 16 illustrates the configuration of the inner plug portion 650. As illustrated in Figure 16, the inner plug portion 650 has, in addition to the leading end portion 651 and the channel 652, a leading end surface 653, an opening 654 of the channel 652, a male threaded portion 655, and a heating unit 656. As illustrated in Figure 15, the inner plug portion 650 is formed such that the outer peripheral surface of the male threaded portion 655 and the outer peripheral surface of the portion that continues to the rear end side (the side of the storage chamber 621) therefrom combines with the coating head 612 by loosely engaging with the inner peripheral surface of the rear end portion of the coating head 612. In this manner, in a state in which the inner plug portion 650 and the coating head 612 are combined, that is, in the state illustrated in Figure 15, the opening 654 of the channel 652 is aligned with the discharge outlet 612a of the coating head 612 (refer to Figure 18). Until the cartridge 620 is accommodated in the main body 610a of the housing 610, a cartridge cap 670 (refer to Figure 15) that engages with the male threaded portion 655 is attached to the inner plug portion 650 of the cartridge 620. As a result, the interior of the inner plug portion 650 is maintained in a substantially hermetically sealed state with respect to the exterior. In addition, the coating head 612 is formed by a metal having favorable thermal conducting properties as a whole, except for the heating unit 656.

When the health and beauty device 6 of the present embodiment is utilized, the threaded cap 610c is removed from the housing 610. As a result, the protrusion 610f exits the channel 652 of the inner plug portion 650, and the channel 652 is opened. The content 25 which is stored in the storage chamber 621 flows out from the discharge outlet 612a of the coating head 612 via the channel 652 of the inner plug portion 650 by the rotatable operating portion 623 being operated. In the present embodiment, since the heating unit 656 (refer to Figure 16) is provided, even if the content 25 is a solid or the like which originally does not exhibit fluidity, it becomes possible to cause the content 25 to flow out from the discharge outlet 612a of the coating head 612 while melting and fluidizing the content 25 with the heating unit 656.

Therefore, it is possible to apply the content 25 that flows out to a body surface of a human body while the surface 612b (refer to Figure 18) of the coating head 612 is in contact with the body surface of the human body. In addition, the heater 14 described above generates heat by being supplied with electrical power in the same manner as in the first embodiment. Thereby, since the surface 612b which is the coating surface of the coating head 612 is heated to a predetermined temperature, a user can experience a pleasant feeling of application when applying the content 25 to the body surface as described above in this case as well. In addition, it is possible to simultaneously and easily perform discharge and application of the content 25. Further, by replacing the cartridge 620, it is possible to select different types of cosmetic materials or the like according to the target of application and or the user's preference, and to easily exchange and utilize such cosmetic materials. The heating unit 656 for melting the content 25 may also function as a temperature operation portion that heats the surface 612b.

Note that the heating unit 656 shown in Figure 16 may be constituted by an electric resistor, for example. The control for maintaining the heating unit 656 as well as the heater 514 illustrated in Figure 10 at a predetermined temperature may be exerted by using the control circuit and the like in the same manner as that described for the first embodiment. Further, instead of the heating unit 656 illustrated in Figure 16, a coil 657 may be provided as illustrated in Figure 17, and this coil 657 may also heat the content 25 or warm the surface 612b by electromagnetic induction heating.

The configuration illustrated in Figure 15 adopts a configuration in which the inner plug portion 650 is integrated with the coating head 612 by engagement. Therefore, it is desirable to provide a mechanism to prevent the inner plug portion 650 or the coating head 612 from rotating together with the rotatable operating portion 623 when the rotatable operating portion 623 is operated to feed the content 25. It is also desirable to provide a mechanism that prevents the position of the inner plug portion 650 from shifting backward (toward the right in Figure 15) relative to the main body 610a of the housing 610.

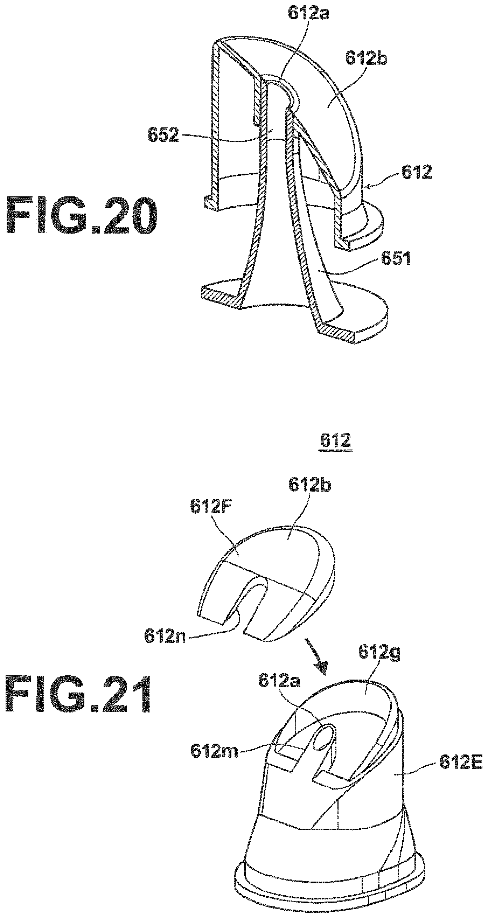

Next, another example of the coating head 612 will be described with reference to Figures 20 through 22. The coating head 612 illustrated in Figure 18 and Figure 19 is formed entirely by metal. In contrast, in the coating head 612 illustrated in Figure 20, a leading end portion 651, formed by a metal, of the inner plug portion 650 enters the discharge outlet 612a. Meanwhile, the surface 612b, which is the coating surface that surrounds the discharge outlet 612a, is formed by a material other than metal, for example, synthetic resin.

Next, the coating head 612 illustrated in Figure 21 is constituted by a lower portion 612E and an upper portion 612F. The upper portion 612F is configured to be fitted into a recess 612g which is formed in the upper portion of the lower portion 612E. The lower portion 612E is provided with a convex portion 612m that includes a discharge outlet 612a, and at least a portion of the discharge outlet 612a is formed by a synthetic resin. The upper portion 612F is formed entirely by a metal and has a concave portion 612n that engages with the convex portion 612m. In the coating head 612 having such a configuration, the part which is the discharge outlet 612a is formed by a synthetic resin, while the surrounding surface 612b is formed by a metal.

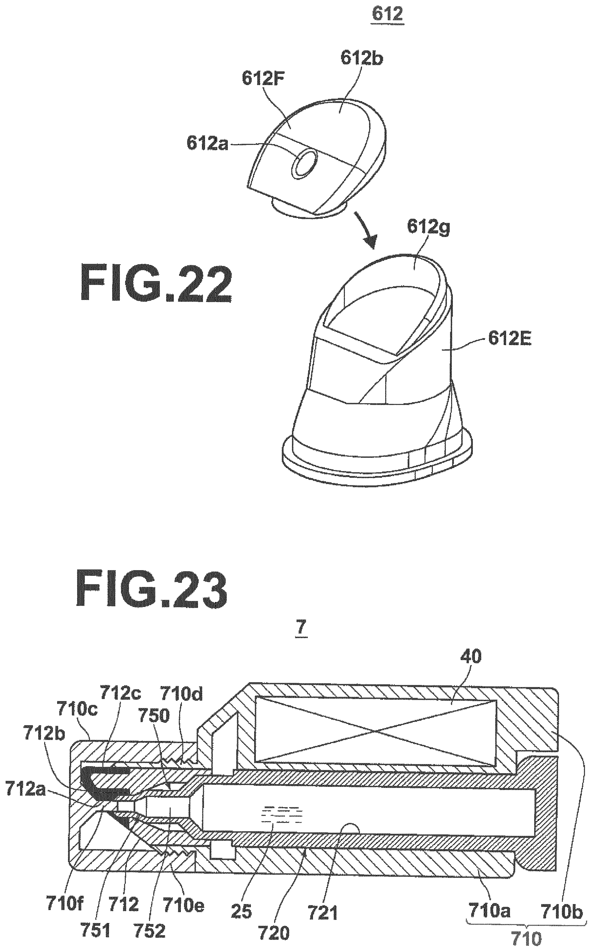

Next, the coating head 612 illustrated in Figure 22 is constituted by a lower portion 612E and an upper portion 612F in the same manner as described above. The upper portion 612F is formed entirely by a metal and is configured to be fitted into a recess 612g which is formed in the upper portion of the lower portion 612E. A discharge outlet 612a is provided in the upper portion 612F at a position shifted from the center of an inclined surface 612b (a position below the inclination). In such a configuration, the portion of the surface 612b which is shifted from the discharge outlet 612a may be widely utilized as a coating surface. The portion of the surface 612b which is shifted from the discharge outlet 612a in this manner is effective in preventing contamination by the content, and also provides a distinctive appearance to enhance the commercial value of the health and beauty device. Note that the inclination of the surface 612b described above means that the surface 612b is inclined with respect to the outflow direction of the content (the direction from the lower side to the upper side in Figure 22). In the case that the discharge outlet 612a is formed in the inclined surface 612b, the content that flows out from the discharge outlet 612a naturally bends and spreads toward the upper side of the inclination. Therefore, in the case that the discharge outlet 612a is provided at a position shifted from the center of the inclined surface 612b, it is for the discharge outlet 612a to be provided at a position at the lower side of the inclination as in the present example, such that the content spreads toward the wide upper portion of the surface 612b.

The configurations of the peripheries of the discharge outlets 612a and the surfaces 612b of the coating heads 612 of Figures 18 through 22 described above are not limited to the sixth embodiment in particular, and may be applied to the other embodiments as appropriate.

Next, a seventh embodiment of the present invention will be described. Figure 23 is a side sectional view showing a health and beauty device 7 according to the seventh embodiment of the present invention. The health and beauty device 7 of the present embodiment includes a housing 710 constituted by a cartridge housing portion 710a and a battery housing portion 710b, a threaded cap 710c which is capable of being attached to the housing 710, and a cartridge 720 which is removably accommodated in the cartridge housing portion 710a. The cartridge housing portion 710a and the battery housing portion 710b are disposed in a state in which their respective longitudinal directions are aligned to be parallel.

The cartridge 720 has a substantially cylindrical storage chamber 721 that stores the content 25, a rotatable operating portion 723, and an inner plug portion 750 which is connected to the storage chamber 721. A substance having fluidity is employed as the content 25 in the present embodiment. The rotatable operating portion 723 is coupled to a mechanism for feeding the content 25 from the storage chamber 721, that is, the mechanism constituted by the piston 27, the threaded rod 28, etc. illustrated in Figure 1. However, this mechanism is omitted from Figure 23. The inner plug portion 750 has a leading end portion 751 with a thin cylindrical portion. A channel 752 that communicates with the storage chamber 721 and extends to the interior of the leading end portion 751 is formed within the inner plug portion 750.

Note that in the cartridge 720 of the present embodiment as well, it is desirable for a cartridge cap similar to the cartridge cap 670 illustrated in Figure 15 to be attached such that the interior and the exterior of the inner plug portion 750 are maintained in a hermetically sealed state until the cartridge 720 is utilized.

An obliquely cut cylindrical leading end portion 710d is formed in the cartridge housing portion 710a of the housing 710. A coating head 712 is attached to the interior of the leading end portion 710d as a coating portion. The coating head 712 has a discharge outlet 712a and a surface 712b which are respectively similar to the outlet 612a and the surface 612b of the sixth embodiment. In other words, the surface 712b is formed flat so as to be a coating surface for applying the content 25 to a body surface of a human body. The discharge outlet 712a, through which the content 25 flows out, is formed in the surface 712b. At least a portion 712c, which is colored black in Figure 23, of the coating head 712 is formed by a metal having favorable thermal conducting properties, such as aluminum, an aluminum alloy, and stainless steel, for example. The surface 712b of the coating head 712 includes the surface of the metal portion 712c. A heater (not shown) similar to the heater 14 illustrated in Figure 1 and Figure 15 is fixed to be in close contact with the metal portion 712c at the back surface of the surface 712b.

In the case that the cartridge cap described above is attached, the cartridge 720 is utilized after the cartridge cap is removed. The cartridge 720 is accommodated in the cartridge housing portion 710a with the inner plug portion 750 entering the interior space of the coating head 712. In this state, the leading end portion 751 of the inner plug portion 750 loosely engages the cylindrical portion formed in the metal portion 712c of the coating head 712. At this time, the position of the cartridge 720 in the longitudinal direction thereof relative to the coating head 712 (the position in the horizontal direction in Figure 23) is determined by, for example, bringing a part of the cartridge 720 into contact with a rib (not shown) provided in the cartridge housing portion 710a. When the cartridge 720 is accommodated in the cartridge housing portion 710a in this state, the channel 752 of the inner plug portion 750 communicates with the discharge outlet 712a of the coating head 712. It is desirable for a sealing member constituted by a contact ring, an O ring, or the like to be provided between the leading end portion 751 and the cylindrical portion of the coating head 712, in order to prevent the channel 752 within the leading end portion 751 from communicating with a space other than the discharge outlet 712a of the coating head 712.

After the cartridge 720 is accommodated in the cartridge housing portion 710a of the housing 710, a threaded cap 710c is attached to the housing 710 when the health and beauty device 7 is not being utilized. The threaded cap 710c is formed in a cylindrical shape with one closed end. The threaded cap 710c is attached to the housing 710 by threading female threads formed on the open end 710e thereof onto a male threaded portion formed on the leading end portion 710d. In addition, a columnar protrusion 710f is provided on the inner surface of the closed end of the threaded cap 710c. In a state in which the threaded cap 710c is attached to the housing 710, the protrusion 710f enters the channel 752 of the leading end portion 751 of the inner plug portion 750. Thereby, the channel 752 is maintained in hermetically sealed state with respect to the exterior of the cartridge 720.

When the health and beauty device 7 of the present embodiment is utilized, the threaded cap 710c is removed from the housing 710. As a result, the protrusion 710f exits the channel 752 of the inner plug portion 750, and the channel 752 is opened. The fluid content 25 which is stored in the storage chamber 721 flows out from the discharge outlet 712a of the coating head 712 via the channel 752 of the inner plug portion 750 by the rotatable operating portion 723 being operated.

Therefore, it is possible to apply the content 25 that flows out to a body surface of a human body while the surface 712b of the coating head 712 is in contact with the body surface of the human body. In addition, the heater described above generates heat by being supplied with electrical power in the same manner as in the first embodiment. Thereby, since the surface 712b which is the coating surface of the coating head 712 is heated to a predetermined temperature, a user can experience a pleasant feeling of application when applying the content 25 to the body surface as described above in this case as well. In addition, it is possible to simultaneously and easily perform discharge and application of the content 25. Further, by replacing the cartridge 720, it is possible to select different types of cosmetic materials or the like according to the target of application and or the user's preference, and easily exchange and utilize such cosmetic materials.

In the configuration illustrated in Figure 23, the cartridge 720 is integrated with the coating head 712 by engagement. Therefore, it is desirable to provide a mechanism to prevent the inner plug portion 750 or the coating head 712 from rotating together with the rotatable operating portion 723 when the rotatable operating portion 723 is operated to feed the content 25. It is also desirable to provide a mechanism that prevents the position of the inner plug portion 750 from shifting backward (toward the right in Figure 23) relative to the main body 710a of the housing 710.

The health and beauty device of each of the embodiments described above uses a cartridge storing a lip plumper, which is a lip cosmetic material, as the content. However, the content is not limited thereto. That is, in the health and beauty device of the present invention, any substance may be employed as long as it is content which is applied to the body surface of the human body and imparts at least one of a cosmetic effect and a health effect. In addition to the lip cosmetic material, an eye cosmetic material, a lip gloss, a lip cream, a face cream, an eye cream, a hair color, a hair treatment, and a depilatory cream, for example, may be employed as the content to be applied to the body surface of the human body to give a cosmetic effect. Note that hair which grows on the body surface is also included in the "body surface" as referred to in the present invention.

Meanwhile, a skin disease treating agent, an analgesic anti-inflammatory agent, a disinfectant, an insect sting inhibitor, an anti-itch agent, and the like may be examples of the content applied to the body surface of the human body to impart a health effect.

As the fluid content, a substance in the form of a cream having a high viscosity to a certain degree may be favorably employed, but a liquid substance may also be employed. In the case that a liquid content is employed, it is possible to prevent the content from flowing out of the outlet while the feeding portion is not operated, by maintaining the outlet which is provided in the cartridge sufficiently small.

In addition, in the embodiments described above, the feeding portions that cause the content to flow out of the cartridge is that which have the rotatable operating portions, but feeding portions for causing the content to flow out of the cartridge by operations other than rotary operation may alternatively be employed. More specifically, a knock type feeding portion equipped with a knock operating portion that moves forward and backward in response to an external knocking force, and a moving body which moves the interior of the cartridge toward the content outflow outlet by the operation of the knock operating portion may be employed. Note that Japanese Unexamined Patent Publication No. 11-225826 discloses an example of such a knock type feeding mechanism.

As a further alternative, a so called squeeze type feeding portion constituted by a flexible member that forms at least a part of the outer shell of the cartridge that applies pressure to the stored content by being deformed under an externally applied squeezing force may be employed.

In the health and beauty device of the present invention, it is also possible to apply a cartridge in which a first end of a tube (tube) formed by an elastic member is in communication with a part of an outer shell that stores content. A second end of the tube opposite the first end is an outlet from which the content flows out, and the entire cartridge constitutes a feeding portion. In the case that a cartridge constituted by such an outer shell and a tube is employed, a pump called a tube pump, a roller pump, an ironing pump, a peristaltic pump or the like may be mounted in the main body of the health and beauty device main body. The content which is stored in the outer shell can be caused to flow out from the second end (outlet) of the tube, by pumping the tube portion with the pump. With respect to a pump such as that described above, a favorable example is disclosed in Japanese Unexamined Patent Publication No. 2013-245665, for example.

In the case that the above tube pump is applied, even if the fluid content is highly viscous, the content can be caused to flow out from the cartridge.

Further, in each of the embodiments described above, the temperature operation portion incorporated in the coating portion is constituted by a heater. Alternatively, the temperature operation portion may be constituted a cooling member for cooling the body surface of the human body. Such a cooling member may be configured by employing a Peltier element, for example. Applying cosmetic materials and the like while cooling the body surface with the cooling member also enables a pleasant feeling of application to be provided to a user especially in an environment with a high temperature.

Further, the power source unit is not limited to the battery (dry cell battery) 40 described above. Alternatively, it is also possible to adopt a power source unit supplied with electrical power from a rechargeable battery or a USB connector used for a personal computer and the like.

### [Explanation of the Reference Numerals]

1, 2, 3, 4, 5, 6, 7 health and beauty device
10, 210, 310, 410, 510, 610, 710 housing
10a, 510a, 610a main body of housing
10b, 510b, 610b lid of housing
11 recess in housing
12, 212, 312, 412 coating portion
12a, 512a, 612a, 712a discharge outlet of coating portion
12b, 512b, 612b, 712b surface of coating portion (coating surface)
13 switch
14, 514 heater
20, 220, 320, 420, 520, 620, 720 cartridge
21,521, 621, 721 storage chamber
22 cylinder chamber
23, 223, 323, 423, 523, 623, 723 rotatable operating portion
25 content
26, 326, 426 outlet
27 piston
28 threaded rod
30 rotary holding portion
31 cover
512, 612, 712 coating head (coating portion)
656 heating unit
657 coil
710a cartridge housing portion of housing
710b battery housing portion of housing

## Claims

1. A health and beauty device comprising:
a cartridge equipped with a storage portion that stores a fluid content which is applied to a body surface of a human body to impart at least one of a cosmetic effect and a health effect, an outlet from which the content flows out, and a feeding portion that causes the content to flow out from the outlet by an externally applied force;
a housing that removably accommodates the cartridge,
a coating portion provided in a part of the housing, having a discharge outlet aligned with the outlet of the cartridge accommodated in the housing and a coating surface for applying the content that flows out of the outlet and the discharge outlet to the body surface of the human body;
a temperature operation portion for heating or cooling the coating surface by receiving electric power; and
a power source unit for supplying the electrical power.

2. A health and beauty device comprising:
a cartridge equipped with a storage portion that stores content which is applied to a body surface of a human body to impart at least one of a cosmetic effect and a health effect, a heating unit that heats the content to impart fluidity thereto, a coating portion having a discharge outlet from which the content flows out and a coating surface for applying the content that flows out of the discharge outlet to the body surface of the human body, and a feeding portion that causes the content to flow out from the discharge outlet by an externally applied force;
a housing that removably accommodates the cartridge,
a temperature operation portion for heating or cooling the coating surface by receiving electric power; and
a power source unit for supplying the electrical power.

3. A health and beauty device as defined in Claim 1 or Claim 2, wherein:
the content is a cosmetic material.

4. A health and beauty device as defined in Claim 3, wherein:
the cosmetic material is a cosmetic material for lips.

5. A health and beauty device as defined in Claim 3, wherein:
the cosmetic material is a cosmetic material for eyes.

6. A health and beauty device as defined in Claim 1 or Claim 2, wherein:
the content is a pharmaceutical agent.

7. A health and beauty device as defined in any one of Claims 1 through 6, wherein:
the feeding portion of the cartridge comprises a rotatable operating portion which rotates upon receipt of an external force and a moving body that moves within the cartridge by the rotatable operating portion being rotated.

8. A health and beauty device as defined in any one of Claims 1 through 6, wherein:
the feeding portion of the cartridge comprises a knock operating portion which moves forward and backward in response to an external knocking force, and a moving body that moves within of the cartridge by operation of the knock operating portion.

9. A health and beauty device as defined in any one of Claims 1 through 6, wherein:
the feeding portion of the cartridge comprises a flexible member that constitutes at least a portion of the outer shell of the cartridge and applies pressure to the stored content by deforming under an externally applied squeezing force.

10. A health and beauty device as defined in any one of Claims 1 through 9, wherein:
the temperature operation portion has a planar portion provided adjacent to the interior of the coating portion.

11. A health and beauty device as defined in any one of Claims 1 through 10, wherein:
both the cartridge and the power source unit are formed as approximate rod shapes; and
the cartridge and the power source unit are arranged in series with each other in the longitudinal direction.

12. A health and beauty device as defined in any one of Claims 1 through 10, wherein:
both the cartridge and the power source unit are formed as approximate rod shapes; and
the cartridge and the power source unit are arranged parallel to each other with a gap therebetween in a direction that intersects with the respective longitudinal directions thereof.

13. A health and beauty device as defined in any one of Claims 1 through 10, wherein:
both the cartridge and the power source unit are formed as approximate rod shapes; and
the power source unit is arranged at a position remote from the cartridge in the longitudinal direction of the cartridge, oriented such that the longitudinal direction of the power source unit intersects the longitudinal direction of the cartridge.

14. A cartridge which is removably accommodated in a housing that constitutes a health and beauty device, comprising:
a storage portion that stores a fluid content which is applied to a body surface of a human body to impart at least one of a cosmetic effect and a health effect;
an outlet from which the content flows out; and
a feeding portion that causes the content to flow out from the outlet by an externally applied force.

15. A cartridge which is removably accommodated in a housing that constitutes a health and beauty device, comprising:
a storage portion that stores content which is applied to a body surface of a human body to impart at least one of a cosmetic effect and a health effect;
a heating unit that heats the content to impart fluidity thereto;
a coating portion having a discharge outlet from which the content flows out and a coating surface for applying the content that flows out of the discharge outlet to the body surface of the human body; and
a feeding portion that causes the content to flow out from the discharge outlet by an externally applied force.
